# EUROPÄISCHE PATENTSCHRIFT

(11) **EP 0 915 711 B1**
(45) Veröffentlichungstag und Bekanntmachung des Hinweises auf die Patenterteilung: **07.11.2001**
(21) Anmeldenummer: 97936603.6
(22) Anmeldetag: 01.08.1997
(51) Int. Cl.: A61L 2/04, A61L 2/06, A61L 2/12, A61L 2/20, A61M 5/24

(54) **VERFAHREN ZUR TERMINALEN STERILISIERUNG VON BEFÜLLTEN SPRITZEN BEI STÜTZDRUCK**
TERMINAL STERILISATION PROCESS FOR FILLED SYRINGES UNDER AN AUXILIARY PRESSURE
PROCEDE DE STERILISATION TERMINALE DE SERINGUES REMPLIES SOUS UNE PRESSION D'APPOINT

(30) Priorität: 02.08.1996 DE 19632402
(43) Veröffentlichungstag der Anmeldung: 19.05.1999
(73) Patentinhaber: SCHERING AKTIENGESELLSCHAFT, 13342 Berlin (DE)
(72) Erfinder: KOLBERG, Reiner, D-13465 Berlin (DE)
(74) Vertreter: Prüfer, Lutz H., Dipl.-Phys.
(86) Internationale Anmeldenummer: DE9701671
(87) Internationale Veröffentlichungsnummer: WO9805366

(56) Entgegenhaltungen:
- WO-A-94/13328
- WO-A-95/00180
- US-A- 1 424 187
- US-A- 4 628 969
- US-A- 5 207 983

## Beschreibung

Die Erfindung betrifft ein Herstellungsverfahren einer vorgefüllten, sterilen Spritze mit einem zylinderförmigen Spritzenkörper mit einem verschließbaren proximalen und einem verschließbaren distalen Ende, mit einer Innenwandung und einer Außenwandung, einem Spritzenauslaßstück am distalen Ende und einem dieses abdichtenden Verschluß und einem in dem Spritzenkörper gleitfähigen Stopfen, der durch einen Stempel bewegbar ist. Ferner betrifft die Erfindung eine Spritze mit einem, eine Innenwandung aufweisenden Spritzenkörper, einem verschließbaren distalen Ende und einem in dem Spritzenkörper gleitfähigen, durch einen Stempel vom proximalen Ende zum distalen Ende hin bewegbaren Stopfen. Diese Spritzen sind bevorzugt für den Einsatz von injizierbaren Diagnostika, insbesondere Kontrastmittel, vorgesehen, die z.B. in Blutgefäße, Organe, Organteile, Höhlungen und andere Gefäße gespritzt werden und dort bildgebende Wirkung entfalten.

In der Publikation EP 0 227 401 wird ein Verfahren zum Herstellen einer gefüllten, terminal sterilisierten Kunststoffspritze beschrieben. Die Spritze weist einen Zylinder mit einem distalen Ende mit einem Spritzenauslaßstück auf. Das Spritzenauslaßstück wird durch einen Verschluß abgedichtet. Die Spritze wird nach dem Befüllen mit einem flexiblen Gummistopfen verschlossen, der in dem Zylinder gleitfähig ist. Das Verfahren beginnt damit. daß Abfallteilchen oder andere Verunreinigungen von dem Verschluß und dem Kolben entfernt werden. Mikrobielle Verunreinigungen auf dem Verschluß und dem Kolben werden zerstört. Der Zylinder wird mit einer Vielzahl von Wasserstrahlen gewaschen, um Pyrogene und Abfallteilchen zu entfernen. Anschließend wird Siliconöl auf die Innenwandung der Spritze aufgetragen. Danach wird der Verschluß auf das Spritzenauslaßstück aufgesteckt. Durch das proximale Ende der Spritze wird das Kontrastmittel in die Spritze eingefüllt. Die Spritze wird anschließend mit dem Stopfen verschlossen. Diese zusammengesetzte und befüllte Spritze wird in einem Autoklaven sterilisiert. Dabei wird neben dem üblichen Autoklavendruck noch ein zusätzlicher Stützdruck in dem Autoklaven erzeugt. Dadurch wird der Druck auf der Außenoberfläche der Spritze gleich oder größer als der Druck auf der Innenoberfläche der Spritze.

Aus der Publikation von Venten und Hoppert (E. Venten und J. Hoppert (1978) Pharm. ind. Vol. 40, Nr 6, Seiten 665 bis 671) ist ein terminales Sterilisieren von vorgefüllten Spritzampullen bekannt. Die Spritzampullen, die einen Stopfen am proximalen Ende aufweisen, werden distal durch den Rollrand befüllt. Der Rollrand wird anschließend durch eine Dichtscheibe abgedichtet, wobei eine Bördelkappe die Dichtscheibe auf dem Rollrand fixiert (M. Junga (1973) Pharm. Ind. Vol. 35, Nr. 11 a, Seiten 824 bis 829). Die vorgefüllten Spritzampullen werden dann in einen Autoklaven überführt, der bezüglich der Temperatur und des Drucks regelbar ist. Damit die Dichtscheibe sich nicht von der Spritzampulle löst, wird in dem Autoklav ein Stützdruck erzeugt. Der Stützdruck wird durch ein zusätzliches Gas aufgebaut. Dadurch ist es möglich, den Druck auf der Innenseite der Dichtscheibe annähernd gleich dem Druck auf der Außenseite der Dichtscheibe zu halten. Hierdurch wird auch eine Bewegung des bereits eingesetzten Kolbens vermieden. Infolge der guten Regelung ist es selbst möglich, Zweikammerspritzampullen, die mit zwei Lösungen gefüllt sind, terminal zu sterilisieren, ohne daß eine unzulässige Stopfenbewegung oder Dichtscheibenundichtigkeit auftritt.

In der EP 0 553 926 wird ein terminales Sterilisationsverfahren für vorgefüllte Spritzen beschrieben, bei dem kein Autoklav verwendet wird, sondern lediglich eine druckfeste Sterilisationskammer zum Einsatz gelangt. In diese Sterilisationskammer wird die distal oder proximal befüllte Spritze eingebracht. Die Kammer wird mittels Heizgas erwärmt. Zugleich sorgt dieses Heizgas auch für einen Druck, der den Druckanstieg in der Spritze kompensieren soll. Um ein Verdampfen von Flüssigkeit, die durch Kunststoff dringen kann, zu vermeiden, wird gegebenenfalls neben dem Heizgas auch Wasserdampf eingebracht. Es wird in dem Schutzrecht dargestellt, daß dieselbe Sicherheit bezüglich der Sterilisation auftritt, wie sie bei einem Autoklaven erzielt werden kann.

Die WO 95/12482 beschreibt ein Verfahren zur Herstellung von vorgefüllten Kunststoffspritzen, die mit einem Kontrastmittel gefüllt sind. Die Spritzen bestehen aus einem Zylinder, einem Spritzenauslaßstück am distalen Ende, welches für einen Kanülenansatz vorbereitet ist. Weiterhin umfaßt die Spritze einen Stopfen, der in dem Zylinder gleiten kann. Er dichtet das proximale Ende der Spritze ab. Die Spritze ist nach einem Verfahren hergestellt worden, daß zu pyrogenfreien Objekten führt. Weiterhin liegen auch keine Partikel mehr vor. Die Spritze wird durch das proximale Ende befüllt, dabei ist das Spritzenauslaßstück mit einem Verschluß abgedichtet. Die befüllte Spritze wird dann mit dem Stopfen verschlossen. Der Partikelstatus der Räumlichkeiten entspricht den Bedingungen der Klasse 100. Nachdem die Spritzenteile aus der Gußform kommen, werden sie mit Gas abgeblasen, um Partikel zu entfernen. Die Spritze wird anschließend gewaschen. Die Spritze wird danach sterilisiert, so daß die Spritze wahlweise weiterverarbeitet, gelagert oder transportiert werden kann.

Der Stopfen, der zuvor beschriebenen Spritzen, besteht üblicherweise aus einem elastischen Gummimaterial. Dieser Stopfen wird in das Innere des bereits vorgefüllten Spritzenkörpers geschoben. Dafür ist es notwendig, daß eine eigens dafür hergestellte Kompressionsvorrichtung den Stopfen in Hinblick auf die Zylinderwandung zusammenpreßt. Bei elastischem Gummimaterial stellt dieses kein Problem dar. Neben diesem sehr flexiblen Gummimaterial sind auch Stopfen geeignet, welche ein Elastizitätsmodul besitzt, welches deutlich größer als das Elastizitätsmodul von Gummi ist.

Aufgabe der Erfindung ist es, ein Herstellungsverfahren einer vorgefüllten, sterilen Spritze der eingangs beschriebenen Art und eine Spritze der eingangs beschriebenen Art zu schaffen, die es ermöglichen, ein eingefülltes Medium ohne Qualitätseinbuße dauerhaft in der Spritze zu halten. Dabei soll insbesondere die Restluftmenge in der Spritze so gering sein, daß eine Behinderung während des Sterilisierens und des Applizierens nicht auftritt.

Diese Aufgabe wird durch das in Patentanspruch 1 gekennzeichnete Herstellungsverfahren und durch die in Patentanspruch 21 gekennzeichnete Spritze gelöst.

Vorteilhaft ist das erfindungsgemäße Verfahren für alle Stopfenformen und Stopfenmaterialien. Durch diese Vorgehensweise wird die Restluft in der gefüllten Spritze minimal gehalten. Dieses hat erhebliche Vorteile sowohl bei einer terminalen Sterilisierung als auch bei der späteren Applikation. Luftreste sind stets hinderlich, wenn ein Spritzeninhalt einem Patienten injiziert werden soll. Restluft in einer Spritze bei termischem Sterilisieren führt immer zu einem erhöhten Druck, wobei die Summe aus dem Partialdruck besteht, der auf den Wasserdampf zurückzuführen ist, und einem Gaspartialdruck, der durch das in der Spritze befindliche Restvolumen des Gases bewirkt wird.

Stopfen für Spritzen können aus sehr unterschiedlichen Materialien sein. Bei kleinem Volumina sind die zuvor genannten gummielastischen Stopfen bevorzugt. Werden jedoch größere Drücke auf den Stopfen während des Applizierens angewendet, kann es zu unerwünschten Deformationen des gummielastischen Stopfens kommen. Daher sind Stopfen aus einem weniger elastischen Material bevorzugt. Mehr bevorzugt sind Stopfen, welche einem relativ unelastischen, plattenförmigen Kern, aus einem Kunststoffmaterial mit einem Überzug aus gummielastischen Material, besitzen. Solche Stopfen können zweistückig ausgelegt sein. Der feste Kern besteht aus einer Zylinderscheibe, welche in einem innen ausgehöhlten gummielastischen Stopfen sitzt. Der gummielastische Teil besitzt eine relativ dünne Wandstärke, welche einen dichten Sitz des Stopfens gegenüber der Spritzeninnenwandung garantiert. Nachteilig ist jedoch, daß wie sonst üblicherweise verwendeten Kompressionsvorrichtungen für Gummistopfen nicht mehr eingesetzt werden können. Entweder reichen die aufgewendeten Kompressionsdrücke nicht mehr aus, oder es kommt zu nicht mehr reversiblen Deformierungen des Stopfens.

Der Begriff Spritze umfaßt die Begriffe Kartusche (großvolumige Spritze mit mindestens 100 ml Volumen), Ampullenspritzen, Einmalspritzen, Einmalspritzampullen, Einwegspritzen, Injektionsampullen, Spritzampullen, spritzfertige Ampulle, Zylinderampulle und Sofortspritze.

Glasspritzen und Kunststoffspritzen sind in der Publikation von M. Junga ausführlich beschrieben. Eine Mischung aus Glas und Kunststoff wird in WO 96/00098 (Anmeldetag 23.6.1995) dargestellt.

Kunststoffe werden ausführlich in Römpp-Chemie-Lexikon, Herausgeber Jürgen Falbe und Manfred Regitz, 9. Auflage, Stuttgart, 1990 auf den Seiten 2398 ff dargestellt. Bevorzugt sind COC, TPX, PP und Polymethylpenten (COC = Cycloolefincopolymer). Diese Kunststoffe sind besonders für den Einsatz bei vorgefüllten, terminal sterilisierten Spritzen geeignet, weil deren hoher Schmelzpunkt von mindestens 130°C eine Dampfsterilisation gemäß dem Standardverfahren bei 121°C zulassen. Darüber hinaus sind die optischen Eigenschaften für eine arzneibuchgemäße, visuelle Inspektion ausreichend.

Prismenwandteile oder Zylinderwandteile sind im wesentlichen flächige Gebilde, die wie Lanze und Stab den Stopfen zusammendrücken und leiten können. Sie können gegebenenfalls eine unregelmäßige äußere Form besitzen. wichtig ist die Funktion, den Gummistopfen zu komprimieren und dabei wenigstens teilweise zu dem Gummistopfen im wesentlichen komplementär ausgebildet zu sein, um einen möglichst gleichmäßigen Druck auf den Stopfen auszuüben.

Die Begriffe proximal und distal definieren sich aus der Sicht des behandelnden Arztes. Am distalen Ende befindet sich das Spritzenauslaßstück, an dem zum Beispiel die Kanüle oder ein Schlauch, der zu einer Kanüle führt, angeschlossen ist. Am proximalen Ende befindet sich der Stopfen, der das Medium durch das distale Ende bei der Applikation drückt. Die Bewegung des Stopfens kann manuel oder auch mechanisch erfolgen. Der Ausdruck Stopfen umfaßt auch Kolben. Für die manuelle Betätigung der Spritze ist es für das Bedienungspersonal hilfreich, wenn die Spritze am proximalen Ende Fingerhalterungen trägt. Dabei weisen die Fingerhalterungen üblicherweise mindestens eine Fläche als Widerlager für den Zeige- und Mittelfinger auf, wobei die Fläche der Fingerhalterung im wesentlichen senkrecht zu der Achse des Spritzenzylinders steht. Bei mechanischen Pumpenvorrichtungen sind verschiedene Modelle bekannt. Eine Spritze trägt dann bevorzugt eine oder mehrere Gerätehalterungen am vorzugsweise proximalen Ende. Besonders gut ist eine solche mechanische Pumpe in der Puplikation EP 0 584 531 (Anmeldetag 21.7.1993) beschrieben. Auch Mischformen aus Fingerhalterung und Gerätehalterung sind möglich.

Die Spritzen sind üblicherweise drehsymmetrisch, lediglich die Fingerhalterung und Gerätehalterung und bisweilen auch das Spritzenauslaßstück weichen von der Symmetrie ab. So kann das Spritzenauslaßstück exzentrisch angeordnet sein. Besonders bevorzugt ist der Luer - Lock, da er ausschließlich bei der Applikation von Kontrastmitteln dann zum Tragen kommt, wenn mechanische Pumpenvorrichtungen eingesetzt werden. Auch bei der manuellen Applikation vermeidet der Luer - Lock und der damit verbundene Schlauch, daß nicht beabsichtigte Bewegungen des Arztes auf die Kanuüle direkt übertragen werden. Weiterhin sind der einfache Luer-Ansatz und auch der Record-Ansatz bekannt. Die proximalen und die distalen Enden der Spritzen müssen verschließbar sein. Das distale Ende wird durch einen Verschluß abgedichtet, der auf das Spritzenauslaßstück aufsetzbar ist. Das Spritzenauslaßstück umfaßt in diesem Schutzrecht die Decke des Spritzenzylinders. Weiterhin umfaßt das Spritzenauslaßstück eine Röhre, die zu der Nadel oder dem Schlauch führt, ein Endstück, welches mit der Nadel oder dem Schlauch in Kontakt steht und einem Zylinder mit Gewinde auf der Innenseite, wobei der Zylinder das Endstück umgibt und ein Gewinde für ein zum Beispiel Luer - Lock trägt. Dabei kann das Spritzenauslaßstück einstöckig oder mehrstöckig sein. Die Decke kann gewölbt eben oder pyramidenförmig sein. Auch Mischformen sind denkbar. Der Stopfen verschließt das proximale Ende der Spritze. Er muß in dem Zylinder gleitfähig sein und muß das Medium sicher von der Umgebung fernhalten. Er soll möglichst wenig für Gase und Flüssigkeiten permeabel sein. Auch Temperaturschwankungen müssen ohne Funktionsstörung aufzufangen sein. Üblicherweise ist der Stopfen bei dem mechanischen Entleeren der Spritze nicht mit einem eigenen Stempel versehen. Vielmehr greift ein Stempel, der Teil der Pumpenvorrichtung ist, in einen Verschluß im Inneren des Stopfens ein, so daß eine Bewegung des Stopfens problemlos möglich ist (Vergleiche Publikation EP 0 584 531). Der Stopfen kann gerade bei der mechanischen Applikation aus einem Material sein, welches weniger elastisch ist als das sonst übliche Gummimaterial. Derartige Stopfen sind notwendig, da der Druck, welcher durch die Pumpenvorrichtungen hervorgerufen wird, zu einer Deformation des sonst üblichen Gummimaterials führt. Das sonst übliche Gummimaterial weicht den auftretenden Belastungen aus, es kommt zu nicht gewünschten Undichtigkeiten zwischen Gummistopfen und Spritzeninnenwandung. Derartige Fehler können die gesamte Applikationsform hochgradig gefährden. Das Volumen und ebenfalls der auftretende Druck sind nicht mehr klar definiert. Besonders bevorzugt ist dieses Verfahren bei Stopfen, welche nicht mehr einstückig ausgebildet sind. Solche Stopfen besitzen eine Zylinderscheibe aus einem relativ wenig elastischen Material und einem darübergestülpten Dichtungsteil, welches aus zum Beispiel einem gummielastischen Material entsteht. Bei der Verwendung dieses zweiteiligen Stopfens oder eines Stopfens mit ähnlicher wenig elastischer Funktion ergibt sich die Schwierigkeit, mit herkömmlichen Kompressionsvorrichtungen den Gummistopfen zusammenzudrücken. Dadurch verbleibt ein nicht gewünschtes Restvolumen an Luft in der Spritze. Diese Luft kann nicht mehr ohne Weiteres bei dem Einführen des Stopfens in den Spritzenkörper entweichen.

Bevorzugt ist ein Herstellungsverfahren, bei dem das Befüllen der Spritze und Einsetzen des Stopfens zeitlich so geartet ist, daß der Stopfen nach dem Befüllen eingesetzt wird.

Ein anderes bevorzugtes Herstellungsverfahren besteht darin, daß das Befüllen der Spritze und Einsetzen des Stopfens zeitlich so geartet ist, daß der Stopfen vor dem Befüllen eingesetzt wird, wobei
die Spritze durch die Kompressionsvorrichtung befüllt wird,
dabei umfasst die Kompressionsvorrichtung mindestens einen Kanal für die Befüllung und einen Kanal für die Entlüftung.

Ebenfalls ist ein Herstellungsverfahren möglich, bei dem eine Teilbefüllung so erfolgt, daß der Stopfen nach dem Befüllen eingesetzt wird und eine Restbefüllung so vorgenommen wird, daß der Stopfen vor dem Restbefüllen eingesetzt wird, wobei die Spritze durch die Kompressionsvorrichtung bei dem Restbefülien befüllt wird, dabei umfaßt die Kompressionsvorrichtung mindestens einen Kanal für die Befüllung und einen Kanal für die Entlüftung.

Bevorzugt ist ebenfalls ein Herstellungsverfahren, bei dem die Kompressionsvorrichtung aus mindestens einer Nadel zum Befüllen und einer Nadel zum Entlüften besteht. Mehr bevorzugt ist eine Kompressionsvorrichtung, die je zwei Nadeln zum Befüllen und zum Entlüften aufweist.

Mehr bevorzugt ist ein Herstellungsverfahren, wobei die Nadeln an einer Halterung angeordnet sind und die Nadelansatzstellen im wesentlichen auf einer Kreislinie liegen, die sich oberhalb des Kontaktbereichs des Stopfens befindet. Bevorzugter ist eine kreisförmig oder Teilkreisförmig ausgebildete Halterung. Besonders bevorzugt ist ein Herstellungsverfahren, bei dem die Halterung mindestens eine Versorgungsleitung umfaßt. Daneben ist es auch möglich, mit mindestens zwei Versorgungsleitungen zu arbeiten, wobei eine Versorgungsleitung zum Befüllen und die andere zum Entüften dient.

Weiterhin bevorzugt ist ein Herstellungsverfahren bei dem zwischen Spritze und Halterung ein Führungsring angeordnet ist, dessen Ebene paralell zur Ebene der Halterung verläuft. Ein solcher Führungsring erleichert es, die Nadeln sicher in den Spritzenkörper einzuführen. Ein Verkanten oder Blockieren wird dadurch praktisch ausgeschlossen. Mehr bevorzugt ist ein Herstellunsverfahren, bei dem die Nadeln um einen Winkel von 0,5 bis 2°, mehr bevorzugt von einem 1° von der Achse des Spritzenkörpers abweicht, wobei der dem distalen Ende der Spritze am nächsten liegende Teil der Nadel nach innen weist.

Um ein sicheres Befüllen zu erzielen, ist es sinnvoll, den Befüllungsstand eines Spritzenkörpers zu ermitteln. Hierfür ist ein erfindungsgemäßes Herstellungsverfahren vorteilhaft, bei dem der Befüllungsgrad durch einen Sensor ermittelt wird. Bevorzugt ist ein kapazitiver Sensor. Der Einsatz solcher Sensoren bei Befüllungen hat sich besonders bewährt, da hierbei vorteilhaft ist, daß ein berührungsloses und reproduzierbares Füllstandmessen möglich ist, ohne daß dabei die Spritze oder deren Inhalt beeinflußt wird.

Bevorzugt ist ein Herstellungsverfahren, wobei die Sterilisationskammer ein Autoklav oder Sterilisator, mit Dampf, Heißluft und / oder Mikrowelle ist. Derartige Sterilisationskammern sind ausführlich in dem zitierten Stand der Technik beschrieben. Der zusätzliche Stützdruck, der in der Sterilisationskammer aufgebaut werden kann, ist von besonderem Interesse. Am meisten bevorzugt ist dabei ein Herstellungsverfahren, wobei ein Stützdruck durch ein Gas in der Sterilisationskammer aufgebaut wird, wobei der Druck auf die Außenoberfläche der Spritze gleich, größer oder kleiner als der Druck auf die Innenoberfläche der Spritze ist.

Das Medium in der befüllten Spritze ist eine Mischung aus einem fluiden Medium und mindestens einem Gas. Das Medium kann eine Flüssigkeit, eine Lösung, eine Suspension oder eine Emulsion sein. Diese Erscheinungsformen sind in W. Schröter et al., (1987) Chemie; Fakten und Gesetze, 14. Auflage, Leipzig auf den Seiten 23 ff beschrieben. Bevorzugt ist ein fluides Medium, welches ein Kontrastmittel ist . Sterile und reine Produktionsprozesse sind in DAB 1996 oder Ph. EUR beschrieben.

Gleitmittel dienen dazu, daß der Stopfen ohne größeren Kraftaufwand innerhalb des Zylinders bewegt werden kann. Bevorzugt ist Siliconöl, welches folgende Eigenschaft aufweist: Viskosität von mindestens 1000 cSt; Qualität: Medical grade.

Damit der Stopfen beim Sterilisieren nicht innerhalb des Zylinders wandert, kann es vorteilhaft sein, wenn der Stopfen während des Sterilisierens fixiert ist. Dadurch kann eine Druckdifferenz zwischen dem Volumen in der Spritze und dem Volumen um die Spritze herum aufgebaut werden. Ausführlich wird ein solches Fixieren des Stopfens in der französischen Patentschrift mit der Publikationsnummer 2,258,866 beschrieben, welche am 30.1.1974 eingereicht worden ist.

Eine bevorzugte erfindungsgemäße Ausführungsform ist beispielhaft in den Zeichnungen abgebildet.
Figur 1 zeigt einen perspektivischen Schnitt durch einen Spritzenkörper in dem sich ein durch eine Kompressionsvorrichtung eingeführter Stopfen befindet.
Figur 2 zeigt eine Detailansicht des Austritts einer Nadel aus der Halterung.

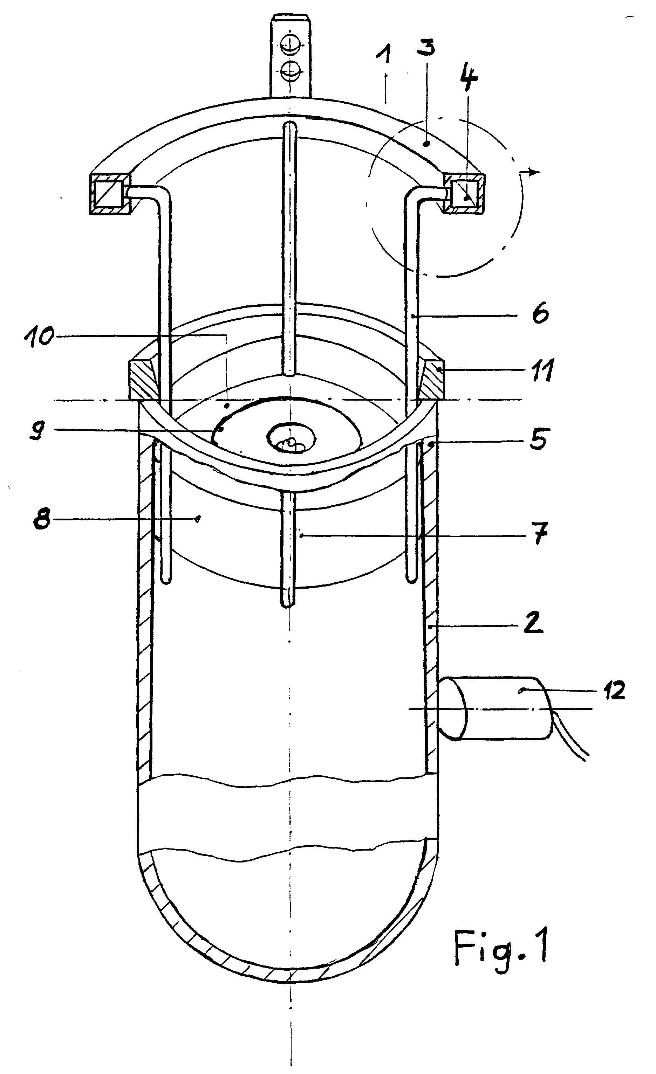

In der Figur 1 wird eine Kompressionsvorrichtung 1 gezeigt, welche in einen Spritzenkörper 2 einer Spritze ragt. Die Kompressionsvorrichtung 1 umfaßt eine Halterung 3, welche ringförmig angeordnet ist und welche eine Versorgungsleitung 4 umfaßt. Die Halterung 3 hat einen rechteckigen Querschnitt. Das Zentrum des Kreisrings der Halterung 3 fällt mit der Achse des Spritzenkörpers zusammen. Die Halterung 3 ist außerhalb des proximalen Endes 5 des Spritzenkörpers 2 angeordnet. Von der Halterung 3 erstrecken sich L-förmig ausgebildete Nadeln 6 in Richtung des Spritzenkörpers 2, wobei der lange Schenkel der Nadeln 6 parallel zu der Achse des Spritzenkörpers verläuft. Die Nadeln 6 berühren mit dem langen Schenkel die Innenwandung 7 des Spritzenkörpers 2. Ebenfalls berühren die langen Schenkel der Nadeln 6 den Stopfen 8, welcher zweistückig ausgebildet ist. Der Stopfen 8 besitzt einen festen Kern 9, welcher wenig elastisch ist. Der Kern 9 weist im wesentlichen die Form einer Scheibe auf, die im Zentrum ein Loch besitzt, in dem ein in der Zeichnung nicht abgebildeter Stempel befestigbar ist. Der Kern 9 des Stopfens 8 ist von einem Stopfenmantel 10 umgeben, welcher den Kern 9 fast vollständig umfaßt. Lediglich in den nach proximal weisenden Teil des Kolbens 8 umfaßt der Mantel 10 nicht den Kern 9. Der Mantel 10 ist aus einem gummielastischen Material hergestellt. Er sorgt für die Abdichtung gegenüber der Innenwandung 7 des Spritzenkörpers 2. Der Stopfen 8 ist an seinem distalen Ende pyramidenförmig ausgebildet. Er weist eine Form auf, welche komplementär zu dem Spritzenauslaßstück am distalen Ende ist.

Unterhalb der Halterung 3 ist ein Führungsring 11 angeordnet , welcher die langen Schenkel der Nadeln 6 außen berührt. Der Führungsring 11 liegt dem proximalen Ende 5 des Spritzenkörpers 2 an. Der Führungsring hat einen konisch aufgebauten Querschnitt, wobei er im distalen Bereich einen breiteren Querschnitt besitzt als im proximalen Bereich.

Bei dem Befüllen einer Spritze wird zuerst der Führungsring 11 auf das proximale Ende des Spritzenkörpers aufgesetzt. Anschließend fährt die Halterung 3, an der sich die Nadeln 6 befinden von außerhalb der Spritze in Richtung des Spritzenkörpers . Dabei haben die Nadeln 6 bereits den Stopfen 8 ergriffen und den gummielastischen Mantel 10 ein wenig nach innen gedrückt. Die Halterung 3 führt nun den Stopfen einschließlich der Nadeln 6 durch den Führungsring 11 in den Spritzenkörper 2 ein. Dabei wird zugleich durch die Kanäle in den Nadeln Luft aus dem Spritzenkörper entfernt. Wahlweise kann dabei zuvor schon die Spritze mit dem flüssigen Medium gefüllt sein oder erst nach dem Einführen des Stopfens befüllt werden. Wesentlich bei dem Procedere ist, den Stopfen 8 in den Spritzenkörper 2 einzuführen, so daß Restluft zwischen Stopfen 8 und der Flüssigkeit aus dem Spritzenkörper 2 entfernt wird.

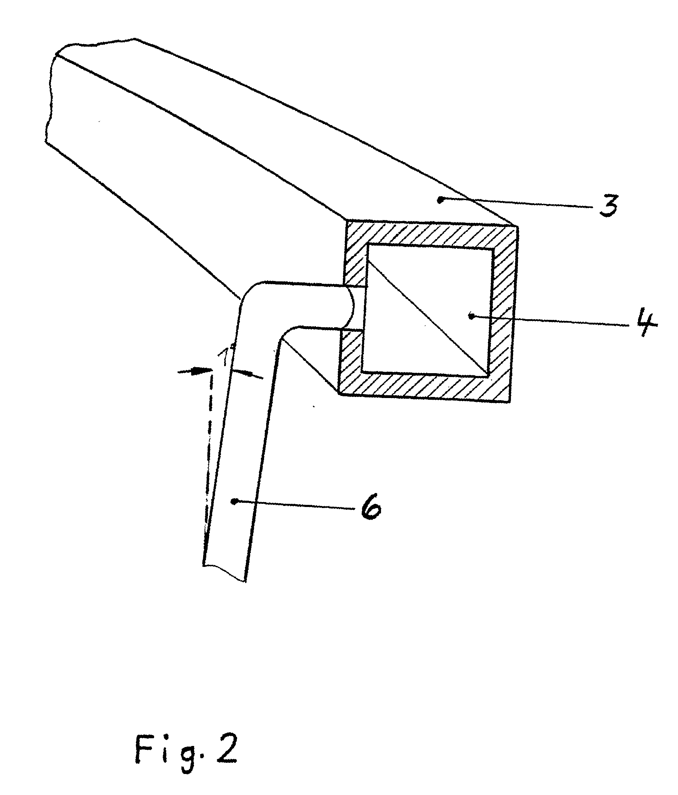

Die Figur 2 zeigt im Detail die Halterung 3, aus der eine Nadel austritt. Hierbei wird besonders deutlich, daß der lange Schenkel der Nadel 6 einen Winkel von 1° gegenüber der Achse des Spritzenkörpers aufweist. Die Versorgungsleitung 4 kann auch wegfallen. Es müßten dann die Nadeln 6 durch die Halterung 3 nach außen geführt werden. Dort würden die Nadeln einzeln an die Versorgungs-Entsorgungsleitung angeschlossen werden.

In der Figur 1 ist ebenfalls ein Sensor 12 abgebildet, welcher die Füllhöhe beim Befüllen registrieren kann. Dieser Sensor 12 ist als kapazitiver Sensor aufgebaut.

### Bezugszeichenliste:

- 1: Kompressionsvorrichtung
- 2: Spritzenkörper
- 3: Halterung
- 4: Versorgungsleitung
- 5: proximale Ende
- 6: Nadel
- 7: Innenwandung
- 8: Stopfen
- 9: Kern
- 10: Mantel
- 11: Führungsring
- 12: Sensor

## Patentansprüche

1. Herstellungsverfahren einer vorgefüllten, sterilen Spritze mit
einem zylinderförmigen Spritzenkörper (2) mit einem verschließbaren proximalen und einem verschließbaren distalen Ende, mit einer Innenwandung (7) und einer Außenwandung,
einem Spritzenauslaßstück am distalen Ende und einem dieses abdichtenden Verschluß,
einem in dem Spritzenkörper (2) gleitfähigen Stopfen (8), der durch einen Stempel bewegbar ist,
wobei das Verfahren die folgenden Schritte umfaßt:
- Bereitstellen des von Keimen, Pyrogenen und/oder Endotoxinen befreiten sowie partikelarmen Spritzenkörpers (2),
- Bereitstellen des von Keimen, Pyrogenen und/oder Endotoxinen befreiten sowie partikelarmen Verschlusses,
- Bereitstellen des von Keimen, Pyrogenen und/oder Endotoxinen befreiten sowie partikelarmen Stopfens (8),
- Auftragen eines Gleitmittels,
- Abdichten des distalen Endes durch den Verschluß des Spritzenauslaßstückes,
- Befüllen der Spritze durch das proximale Ende (5) und Einsetzen des Stopfens (8) durch das proximale Ende (5),
- Entlüften der Spritze durch mindestens partielles Komprimieren des Stopfens (8),
wobei der Kontaktbereich des Stopfens (8), welcher die Innenwandung des Spritzenkörpers (2) kontaktiert, mittels einer Kompressionsvorrichtung (1) eingedrückt wird, welche innen mindestens eine einen Kanal aufweisende Nadel (6), Lanze, einen Stab, Zylinderwandteil oder Prismenwandteil aufweist, wobei der Kanal mit dem Innenraum des Spritzenkörpers unterhalb des Stopfens kommuniziert, und
- thermisches Sterilisieren in einer Sterilisationskammer.

2. Herstellungsverfahren nach Anspruch 1, wobei das Befüllen der Spritze und Einsetzen des Stopfens (8) zeitlich so geartet ist, daß der Stopfen (8) nach dem Befüllen eingesetzt wird.

3. Herstellungverfahren nach Anspruch 1, wobei das Befüllen der Spritze und Einsetzen des Stopfens (8) zeitlich so geartet ist, daß der Stopfen (8) vor dem Befüllen eingesetzt wird,
wobei die Spritze durch die Kompressionsvorrichtung (1) befüllt wird,
dabei umfaßt die Kompressionsvorrichtung (1) mindestens einen Kanal für die Befüllung und einen Kanal für die Entlüftung.

4. Herstellungverfahren nach Anspruch 1, wobei eine Teilbefüllung nach Anspruch 2 erfolgt und eine Restbefüllung nach Anspruch 3.

5. Herstellungsverfahren nach einem der vorherigen Ansprüche 1, 3 und 4, wobei die Kompressionsvorrichtung (1) aus mindestens einer Nadel (6) zum Befüllen und einer Nadel (6) zum Entlüften besteht.

6. Herstellungsverfahren nach Anspruch 5, wobei die Nadeln (6) an einer Halterung (3) angeordnet sind und die Nadelansatzstellen im wesentlichen auf einer Kreislinie liegen, die sich oberhalb des Kontaktbereichs des Stopfens (8) befindet.

7. Herstellungsverfahren nach Anspruch 6, wobei die Halterung (3) mindestens eine Versorgungsleitung umfaßt.

8. Herstellungsverfahren nach Anspruch 6 oder 7, wobei zwischen Spritze und Halterung (3) ein Führungsring angeordnet ist, dessen Ebene parallel zur Ebene der Halterung (3) verläuft.

9. Herstellungsverfahren nach Anspruch 6 bis 8, wobei die Nadeln (6) um einen Winkel von 0,5° bis 2 ° von der Achse des Spritzenkörpers (2) abweicht, wobei der dem distalen Ende der Spritze am nächsten liegende Teil der Nadeln (6) nach innen weist.

10. Herstellungsverfahren nach einem der vorherigen Ansprüche, wobei der Befüllungsgrad durch einen Sensor (13) ermittelt wird.

11. Herstellungsverfahren nach Anspruch 10, wobei der Sensor (13) ein kapazitiver Sensor (13) ist.

12. Herstellungsverfahren nach einem der vorherigen Ansprüche, wobei die Sterilisationskammer ein Autoklav oder Sterilisator, mit Dampf, Heißluft und / oder Mikrowelle ist.

13. Herstellungsverfahren nach einem der vorherigen Ansprüche, wobei ein Stützdruck durch ein Gas in der Sterilisationskammer aufgebaut wird, wobei der Druck auf die Außenoberfläche der Spritze gleich, größer oder kleiner als der Druck auf die Innenoberfläche der Spritze ist.

14. Herstellungsverfahren nach einem der vorherigen Ansprüche, wobei die Spritzen umfassen: Kartuschen, Ampullenspritzen, Einmalspritzen, Einmalspritzampullen, Einwegspritzampullen, Einwegspritzen, Injektionsampullen, Spritzampullen, spritzfertige Ampullen, Zylinderampullen oder Sofortspritzen.

15. Herstellungsverfahren nach einem der vorherigen Ansprüche, wobei der Kunststoff der Polyolefine aus der Gruppe COC, TPX, Polymethylpenten und PP ist.

16. Herstellungsverfahren nach einem der vorherigen Ansprüche, wobei die Spritze einen Luer - Lock am distalen Ende aufweist.

17. Herstellungsverfahren nach einem der vorherigen Ansprüche, wobei das Medium in der befüllten Spritze eine Mischung aus einem fluiden Medium und mindestens einem Gas ist.

18. Herstellungsverfahren nach Anspruch 17, wobei das Medium eine Flüssigkeit, eine Lösung, eine Suspension oder eine Emulsion ist.

19. Herstellungsverfahren nach Anspruch 18, wobei das Medium ein Kontrastmittel ist.

20. Herstellungsverfahren nach einem der vorherigen Ansprüche, wobei der Stopfen (8) während des Sterilisierens fixiert ist.

21. Spritze mit einem eine Innenwandung (7) aufweisenden Spritzenkörper (2), einem verschließbaren distalen Ende und einem in den Spritzenkörper (2) gleitfähigen, durch einen Stempel vom proximalen Ende zum distalen Ende hin bewegbaren Stopfen (8), der in seinem die Innenwandung (7) des Spritzenkörpers (2) kontaktierenden Wandbereich zum Entlüften mittels einer Kompressionsvorrichtung (1) eingedrückt ist, welche innen mindestens eine einen Kanal aufweisende Nadel (6), Lanze, einen Stab, Zylinderwandteil oder Prismenwandteil aufweist, wobei der Kanal mit dem Innenraum der Spritzenkörpers unterhalb des Stopfens kommuniziert.

## Claims

1. Manufacturing process for a pre-filled sterile syringe having
a cylindrical syringe body (2) having a closable proximal end and a closable distal end, having an inner wall (7) and an outer wall,
a syringe outlet piece at the distal end and a closure sealing off the syringe outlet piece,
a stopper (8), which is able to slide in the syringe body (2) and which is movable by means of a driver,
which process comprises the following steps:
- making available the syringe body (2) freed from micro-organisms, pyrogens and/or endotoxins and also having few particles,
- making available the closure freed from micro-organisms, pyrogens and/or endotoxins and also having few particles,
- making available the stopper (8) freed from micro-organisms, pyrogens and/or endotoxins and also having few particles,
- application of a lubricant,
- sealing off the distal end by means of the closure of the syringe outlet piece,
- filling the syringe through the proximal end (5) and inserting the stopper (8) through the proximal end (5),
- venting of the syringe by at least partial compression of the stopper (8),
the contact region of the stopper (8), which contacts the inner wall of the syringe body (2), being pressed in by means of a compression device (1), which on the inside has at least one needle (6), lance, rod, cylindrical wall region or prismatic wall region provided with a channel, the channel being in communication with the internal space of the syringe body below the stopper, and
- heat-sterilising in a sterilisation chamber.

2. Manufacturing process according to claim 1, wherein filling of the syringe and insertion of the stopper (8) are so arranged in time that the stopper (8) is inserted after filling.

3. Manufacturing process according to claim 1, wherein filling of the syringe and insertion of the stopper (8) are so arranged in time that the stopper (8) is inserted before filling,
the syringe being filled through the compression device (1),
the compression device (1) comprising at least one channel for filling and one channel for venting.

4. Manufacturing process according to claim 1, wherein partial filling is carried out in accordance with claim 2 and residual filling in accordance with claim 3.

5. Manufacturing process according to one of the preceding claims 1, 3 and 4, wherein the compression device (1) consists of at least one needle (6) for filling and one needle (6) for venting.

6. Manufacturing process according to claim 5, wherein the needles (6) are arranged on a holder (3) and the needle attachment locations lie essentially on a circular line located above the contact region of the stopper (8).

7. Manufacturing process according to claim 6, wherein the holder (3) comprises at least one supply line.

8. Manufacturing process according to claim 6 or 7, wherein a guide ring is arranged between the syringe and the holder (3), the plane of which guide ring is oriented parallel to the plane of the holder (3).

9. Manufacturing process according to claim 6 to 8, wherein the needles (6) diverge from the axis of the syringe body (2) at an angle of from 0.5° to 2°, that region of the needles (6) which is closest to the distal end of the syringe pointing inwards.

10. Manufacturing process according to one of the preceding claims, wherein the degree of fill is detected by a sensor (13).

11. Manufacturing process according to claim 10, wherein the sensor (13) is a capacitative sensor (13).

12. Manufacturing process according to one of the preceding claims, wherein the sterilisation chamber is an autoclave or steriliser, using steam, hot air and/or microwaves.

13. Manufacturing process according to one of the preceding claims, wherein a ballasting pressure is established in the sterilisation chamber by a gas, the pressure on the outer surface of the syringe being equal to, greater than or less than the pressure on the inner surface of the syringe.

14. Manufacturing process according to one of the preceding claims, wherein the syringes encompass: cartridges, ampoule syringes, single-use syringes, single-use syringe ampoules, disposable syringe ampoules, disposable syringes, injection ampoules, syringe ampoules, ready-for-injection ampoules, barrel ampoules or immediate-use syringes.

15. Manufacturing process according to one of the preceding claims, wherein the plastics is of polyolefins from the group COC, TPX, polymethylpentene and PP.

16. Manufacturing process according to one of the preceding claims, wherein the syringe has a Luer-Lock at the distal end.

17. Manufacturing process according to one of the preceding claims, wherein the medium in the filled syringe is a mixture of a fluid medium and at least one gas.

18. Manufacturing process according to claim 17, wherein the medium is a liquid, a solution, a suspension or an emulsion.

19. Manufacturing process according to claim 18, wherein the medium is a contrast medium.

20. Manufacturing process according to one of the preceding claims, wherein the stopper (8) is fixed during sterilisation.

21. Syringe having a syringe body (2) provided with an inner wall (7), having a closable distal end and having a stopper (8), which is movable by means of a driver from the proximal end towards the distal end and which, in its wall region contacting the inner wall (7) of the syringe body (2), is pressed in, for venting, by means of a compression device (1), which has on the inside at least one needle (6), lance, rod, cylindrical wall region or prismatic wall region having a channel, the channel being in communication with the internal space of the syringe body below the stopper.

## Revendications

1. Procédé de fabrication d'une seringue stérile, pré-remplie, qui est munie d'un corps (2) cylindrique, qui comporte une extrémité proximale et une extrémité distale pouvant être obturées, une paroi intérieure (7) et une paroi extérieure, d'une partie d'évacuation sur l'extrémité distale et d'un obturateur pour fermer hermétiquement celle-ci, d'un bouchon (8) capable de glisser dans le corps de la seringue (2) et qui peut se déplacer par l'intermédiaire d'un piston, le procédé comprenant les étapes suivantes :
- préparation du corps de la seringue (2) exempt de germes, de substances pyrogènes et/ou d'endotoxines et et ne contenant que très peu de particules,
- préparation de l'obturateur exempt de germes, de substances pyrogènes et/ou d'endotoxines et contenant très peu de particules,
- préparation du bouchon (8) exempt de germes, de substances pyrogènes et/ou d'endotoxines et ne contenant que très peu de particules,
- application d'un agent antigrippant,
- réalisation de l'étanchéité de l'extrémité distale par la fermeture de la partie d'évacuation de la seringue,
- remplissage de la seringue par l'extrémité proximale (5) et introduction du bouchon (8) à travers l'extrémité proximale (5),
- évacuation de l'air contenu dans la seringue au moins par une compression partielle du bouchon (8),
la zone de contact du bouchon (8), qui est en contact avec la paroi intérieure du corps de seringue (2), étant introduite au moyen d'un dispositif de compression (1), qui comporte, à l'intérieur, au moins une aiguille (6), munie d'un canal, une lance, une tige, une partie de paroi cylindrique ou une partie de paroi prismatique, le canal communiquant en dessous du bouchon avec le volume intérieur du corps de la seringue, et
- stérilisation thermique dans une chambre de stérilisation.

2. Procédé de fabrication selon la revendication 1, dans lequel le remplissage de la seringue et la pose du bouchon (8) sont effectués chronologiquement, de telle sorte que le bouchon (8) est posé après le processus de remplissage.

3. Procédé de fabrication selon la revendication 1, dans lequel le remplissage de la seringue et la pose du bouchon (8) sont effectués chronologiquement, de telle sorte que le bouchon (8) est posé avant le processus de remplissage, la seringue étant remplie au moyen du dispositif de compression (1), sachant qu'à cet effet, le dispositif de compression (1) comporte au moins un canal pour le remplissage et un canal pour l'évacuation de l'air.

4. Procédé de fabrication selon la revendication 1, dans lequel il est prévu d'effectuer un remplissage partiel selon la revendication 2 et un remplissage du volume restant selon la revendication 3.

5. Procédé de fabrication selon l'une des revendications précédentes 1, 3 et 4, dans lequel le dispositif de compression (1) est formé par au moins une aiguille (6) pour le remplissage et une aiguille (6) pour l'évacuation de l'air.

6. Procédé de fabrication selon la revendication 5, dans lequel les aiguilles (6) sont montées contre un support (3) et les zones de mise en contact des aiguilles sont situées sensiblement sur un cercle, qui se situe au-dessus de la zone de contact du bouchon (8).

7. Procédé de fabrication selon la revendication 6, dans lequel le support (3) comprend au moins un tube d'alimentation.

8. Procédé de fabrication selon la revendication 6 ou 7, dans lequel un anneau de guidage, situé dans un plan parallèle au plan du support (3), est disposé entre la seringue et le support (3).

9. Procédé de fabrication selon l'une quelconque des revendications 6 à 8, dans lequel les aiguilles (6) s'écartent de l'axe du corps de seringue (2) en formant un angle de 0,5° à 2°, la partie des aiguilles (6) la plus proche de l'extrémité distale de la seringue étant orientée vers l'intérieur.

10. Procédé de fabrication selon l'une quelconque des revendications précédentes, dans lequel le degré de remplissage est déterminé par un capteur (13).

11. Procédé de fabrication selon la revendication 10, dans lequel le capteur (13) est un capteur (13) capacitif.

12. Procédé de fabrication selon l'une quelconque des revendications précédentes, dans lequel la chambre de stérilisation est un autoclave ou un stérilisateur à vapeur, à air chaud et/ou à micro-ondes.

13. Procédé de fabrication selon l'une quelconque des revendications précédentes, dans lequel une pression de support est générée par un gaz dans la chambre de stérilisation, la pression sur la surface extérieure de la seringue étant égale, supérieure ou inférieure à la pression sur la surface intérieure de la seringue.

14. Procédé de fabrication selon l'une quelconque des revendications précédentes, dans lequel les seringues comprennent : des cartouches, des seringues à ampoules, des seringues à usage unique, des ampoules pour seringues à usage unique, des ampoules pour seringues non réutilisables, des seringues non réutilisables, des ampoules d'injection, des ampoules pour seringues, des ampoules prêtes à l'injection, des ampoules cylindriques ou des stylos injecteurs.

15. Procédé de fabrication selon l'une quelconque des revendications précédentes, dans lequel la matière plastique des polyoléfines est issue du groupe COC, TPX, polyméthypentène et polypropylène.

16. Procédé de fabrication selon l'une quelconque des revendications précédentes, dans lequel la seringue est munie d'un embout luer-lock sur l'extrémité distale.

17. Procédé de fabrication selon l'une quelconque des revendications précédentes, dans lequel le milieu contenu dans la seringue remplie est un mélange formé par un milieu fluide et au moins un gaz.

18. Procédé de fabrication selon la revendication 17, dans lequel le milieu est un liquide, une solution, une suspension ou une émulsion.

19. Procédé de fabrication selon la revendication 18, dans lequel le milieu est un agent de contraste.

20. Procédé de fabrication selon l'une quelconque des revendications précédentes, dans lequel le bouchon (8) est fixé pendant la stérilisation.

21. Seringue comprenant un corps de seringue (2) muni d'une paroi intérieure (7), une extrémité distale pouvant être obturée et un bouchon (8) capable de glisser pouvant être déplacé dans le corps de la seringue (2) de l'extrémité proximale vers l'extrémité distale au moyen d'un piston, lequel bouchon, en vue d'évacuer l'air, est introduit avec sa paroi en contact avec la paroi intérieure (7) du corps de la seringue (2), au moyen d'un dispositif de compression (1), qui comporte à l'intérieur au moins une aiguille (6) munie d'un canal, une lance, une tige, une partie de paroi cylindrique ou une partie de paroi prismatique, le canal communiquant en dessous du bouchon avec le volume intérieur du corps de la seringue.
